(19) Europäisches Patentamt
European Patent Office
Office européen des brevets

(11) Veröffentlichungsnummer: **0 354 420**
**A1**

(12) # EUROPÄISCHE PATENTANMELDUNG

(21) Anmeldenummer: **89113928.9**

(22) Anmeldetag: **28.07.89**

(51) Int. Cl.⁴: **C07C 51/58 , C07C 59/135 , C07C 53/50**

(30) Priorität: **06.08.88 DE 3826808**

(43) Veröffentlichungstag der Anmeldung: **14.02.90 Patentblatt 90/07**

(84) Benannte Vertragsstaaten: **BE DE FR GB IT NL**

(71) Anmelder: **HOECHST AKTIENGESELLSCHAFT**
**Postfach 80 03 20**
**D-6230 Frankfurt am Main 80(DE)**

(72) Erfinder: **Blickle, Peter, Dr.**
**Hattersheimer Strasse 14**
**D-6233 Kelkheim (Taunus)(DE)**
Erfinder: **Hintzer, Klaus, Dr.**
**Rupertusstrasse 3**
**D-8269 Burgkirchen(DE)**
Erfinder: **Schwertfeger, Werner, Dr.**
**Erlenstrasse 3**
**D-6306 Langgöns(DE)**
Erfinder: **Strutz, Heinz, Dr.**
**Johannesallee 14**
**D-6230 Frankfurt am Main 80(DE)**

(54) **Verfahren zur Herstellung von fluorierten Carbonsäurefluoriden.**

(57) Fluorierte Carbonsäurefluoride der Formel I

$$R - [-O - \underset{|}{\overset{CF_3}{C}}F - CF_2]_n - CF_2 - COF \qquad (I),$$

können aus fluorierten Vinylethern der Formel II

$$R - [-O - \underset{|}{\overset{CF_3}{C}}F - CF_2]_n - O - CF = CF_2 \qquad (II),$$

hergestellt werden, indem die Vinylether auf eine Temperatur von 100 bis 350 °C erhitzt werden.
Aus den Carbonsäurefluoriden können durch Folgereaktionen, beispielsweise Verseifung, Veresterung oder Aminolyse, perfluorierte Carbonsäuren und deren Derivate hergestellt werden.

EP 0 354 420 A1

## Verfahren zur Herstellung von fluorierten Carbonsäurefluoriden

Die Erfindung bezieht sich auf die Herstellung von fluorierten Carbonsäurefluoriden aus fluorierten Vinylethern.

Fluorierte Carbonsäuren und deren Derivate finden in der Technik vielseitige Verwendungsmöglichkeiten. Die Salze langkettiger perfluorierter Carbonsäuren werden als Emulgatoren bei der Herstellung von Polytetrafluorethylen eingesetzt, während fluorierte Carbonsäuren in Form ihrer Säurefluoride für die Synthese von fluorierten Vinylethern benötigt werden. Fluorierte Carbonsäuren werden in die entsprechenden Kolbe-Produkte umgewandelt, die als Inertflüssigkeiten bzw. als Lösemittel für fluorierte Harze dienen und perfluorierte Carbonsäurefluoride werden durch Belichtung in perfluorierte Inertflüssigkeiten überführt.

Durch Reaktionen von fluorierten Säurefluoriden mit fluorierten Epoxiden, beispielsweise Hexafluorpropenoxid (HFPO) oder Tetrafluorethylenoxid (TFEO) entstehen fluorierte Carbonsäurefluoride, die Ethergruppen enthalten. Beim Einsatz von HFPO sind die Produkte immer verzweigt, während TFEO eine schwer herstellbare und handhabbare Substanz ist (vgl. H. Millauer et al., Angew. Chem., Int. Ed. Engl. 24 (1985) 161;

P. Tarrant et al., Fluor.Chem.Rev., 5 (1971) 77).

In der Literatur ist weiterhin die thermische Umlagerung des Perfluormethylvinylethers $CF_3$-O-CF = $CF_2$ zu Perfluorpropionsäurefluorid beschrieben:

$CF_3\text{-O-CF}=CF_2 \xrightarrow{\Delta} CF_3\text{-}CF_2\text{-COF}$.

Die Reaktion wird mit einer kleinen Menge an Vinylether in der Gasphase bei einer Temperatur von 595 °C durchgeführt und liefert eine Ausbeute von 67 % an Säurefluorid, bezogen auf den umgesetzten Vinylether (vgl. R.N. Haszeldine et al., J. Chem. Soc. (C), 1968, 398).

Kurzkettige Perfluoracylfluoride lassen sich bisher nur mit Hilfe von Metallfluoriden als Katalysatoren bei höheren Temperaturen herstellen. Dies trifft für das Perfluorpropionylfluorid und Perfluorbutyrylfluorid zu (vgl. EP-A-0 260 713).

Es bestand nun die Aufgabe, ein Verfahren zur Herstellung von geradkettigen und verzweigten perfluorierten Carbonsäuren und deren Derivaten zu finden. Das Verfahren soll von technisch genutzten Ausgangsmaterialien ausgehen und Verbindungen unterschiedlicher Struktur durch Verwendung nur eines Reaktionstyps ohne Verwendung eines Katalysators zu liefern imstande sein.

Es wurde gefunden, daß fluorierte Vinylether durch Erhitzen auf eine Temperatur von 100 bis 350 °C fluorierte Carbonsäurefluoride ergeben, die ihrerseits in fluorierte Carbonsäuren und deren Derivate überführt werden können.

Die Erfindung betrifft somit das Verfahren gemäß den Ansprüchen.

Fluorierte Carbonsäurefluoride der Formel I

$$R\text{—}[\text{-O - }\underset{}{\overset{CF_3}{\overset{|}{CF}}}\text{ - }CF_2]_n\text{ - }CF_2\text{ - COF} \qquad (I),$$

worin R ein verzweigter oder unverzweigter perfluorierter Rest mit 1-10, vorzugsweise 1-7 Kohlenstoffatomen bedeutet, in dem ein oder mehrere Fluoratome durch andere Halogenatome oder Wasserstoff oder eine andere funktionelle Gruppe, beispielsweise $COOR^4$ ($R^4$ = Alkyl), -$SO_2$F und -CN substituiert sein können und n eine ganze Zahl von null bis 10, vorzugsweise von eins bis 5, insbesondere 1 oder 2 ist, werden aus Vinylethern der Formel II

$$R\text{ - }[\text{-O - }\overset{CF_3}{\overset{|}{CF}}\text{ - }CF_2]_n\text{ - O - CF = }CF_2 \qquad (II),$$

mit R und n gemäß Formel I, durch Erhitzen auf eine Temperatur von 100 bis 350 °C, vorzugsweise 150 bis 300 °C, hergestellt.

Die Umsetzung des Vinylethers der Formel II erfolgt vorzugsweise in einem druckfesten Gefäß, beispielsweise in einem Autoklaven unter Eigendruck oder Normaldruck, vorzugsweise unter Eigendruck. Wegen der Gefahr einer explosionsartigen Reaktion der Substanzen mit Sauerstoff wird unter Inertgasatmosphäre, vorzugsweise Stickstoff oder Argon gearbeitet. Die Reaktion kann auch in einem inerten, halogenier-

ten Lösemittel, wie beispielsweise $CF_2Cl\text{-}CFCl_2$ und $CCl_4$ oder perfluorierten Ethern durchgeführt werden.

Der Fortgang der Reaktion wird durch Entnahme von Proben und Untersuchung dieser Proben durch Gaschromatographie oder Infrarotspektralanalyse überwacht.

Nach der Reaktion können die Produkte durch Folgereaktionen, beispielsweise Verseifung, Veresterung oder Aminolyse, vorzugsweise Veresterung, in ihre entsprechenden Carbonsäuren oder deren Derivate, beispielsweise Ester und Amide, vorzugsweise Ester, überführt.

Die erfindungsgemäße Reaktion ermöglicht es, fluorierte Säurefluoride oder Ketone mit unterschiedlichen Substituenten nach folgenden Schemen um zwei C-Atome zu verlängern:

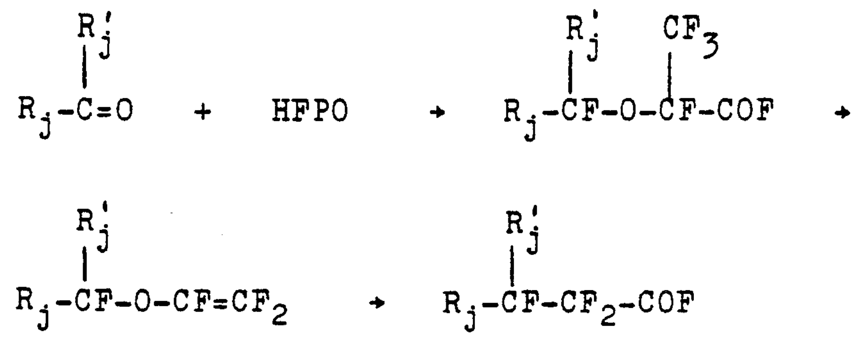

mit $R_j$, $R_j'$ = F, fluorierter Rest.

## Beispiele

### Beispiel 1

In einem 200 $cm^3$ Autoklaven aus rostfreiem Stahl wurden 150 g $CF_3\text{-}CF_2\text{-}CF_2\text{-}O\text{-}CF=CF_2$ vorgelegt. Anschließend wurde der Autoklav mit Stickstoff gespült und 6 Stunden auf 200-230 °C erhitzt.
Nach dem Abkühlen des Autoklaven auf Zimmertemperatur wurden unter Eiskühlung 30 $cm^3$ Methanol in den Autoklaven gegeben. Nach Waschen des Autoklaveninhalts mit Wasser erhielt man 141 g organische Phase, die nach dem Gaschromatogramm (WLD) 68 % $CF_3\text{-}CF_2\text{-}CF_2\text{-}CF_2\text{-}COOCH_3$ enthielten.

### Beispiel 2

In der unter Beispiel 1 beschriebenen Apparatur wurde ein Gemisch aus 80 $cm^3$ $CF_2Cl\text{-}CFCl_2$ und 80 g $CF_3\text{-}CF_2\text{-}CF_2\text{-}O\text{-}CF=CF_2$ 4 Stunden auf 225 °C erhitzt. Das Produktgemisch wurde anschließend mit Methanol verestert und mit Wasser gewaschen. Das Gaschromatogramm des erhaltenen Gemisches zeigte
66 % $CF_2Cl\text{-}CFCl_2$
21 % $CF_3\text{-}CF_2\text{-}CF_2\text{-}CF_2\text{-}COOCH_3$
5 % $CF_3\text{-}CF_2\text{-}CF_2\text{-}O\text{-}CF=CF_2$ .

Die Destillation des Esters ergab eine Ausbeute von 30 g, dabei hatte der Ester einen Siedepunkt von 102-106 °C.

### Beispiel 3

Im Autoklaven des Beispiels 1 wurden 150 g

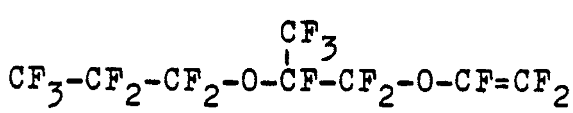

3 Stunden auf 200-215 °C erhitzt.

Der Autoklaveninhalt (149 g) wurde destilliert. Man erhielt bei einem Siedepunkt von 101-105 °C 68 g

einer farblosen Flüssigkeit, die nach dem $^{19}$F-NMR-Spektrum zu 90 % aus

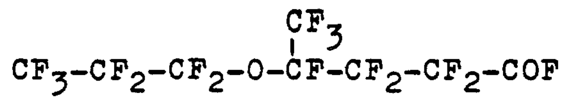

bestand.
Als weitere Komponente wurde der eingesetzte Vinylether nachgewiesen.
Die Ausbeute des Säurefluorids betrug 41 %.

**Beispiel 4**

In einem Glaskolben, der mit einem Magnetrührer, Thermometer, Einleitungsrohr mit Fritte, Füllkörperkolonne und einem Kolonnenkopf versehen war, wurden 130 ml des Perfluorpolyethers

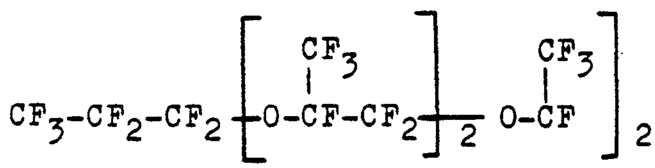

so vorgelegt, daß die Fritte vollständig eintauchte. Anschließend wurde die Apparatur auf eine Innentemperatur von 215 bis 225 °C erhitzt. Mit einer Dosierpumpe setzte man innerhalb von 7 Stunden 89 g des Ethers, der in Beispiel 3 beschrieben wurde, hinzu.
Die Destillation des Produktes ergab eine Ausbeute von 66 g. Eine Probe des Destillats wurde mit Methanol verestert und gaschromatographisch (WLD) untersucht. Das Produkt enthielt:

27,8 % 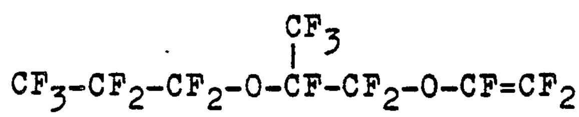

und 66,4 % 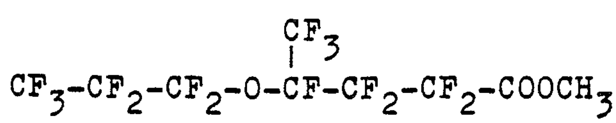

Diese Verfahrensweise eignet sich zur kontinuierlichen Umlagerung von Vinylethern im Säurefluorid.

**Beispiel 5**

Entsprechend Beispiel 2 wurden 80 g des Vinylethers aus Beispiel 3 in 80 $cm^3$ $CF_2Cl$-$CFCl_2$ innerhalb von 10 Stunden bei 200 °C umgesetzt.
Das Produkt wurde anschließend mit Methanol verestert und destilliert.
Die Ausbeute an

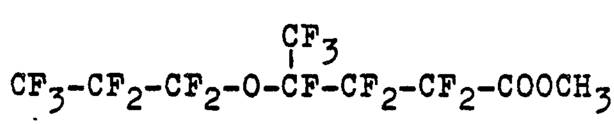

betrug 42 % (35 g) bei einem Siedepunkt von 58-62 °C/26 mbar.

**Beispiel 6**

In einem NMR-Rohr wurde ein Gemisch aus 91 Gew.-% $(CF_3)_2CF$-O-CF = $CF_2$ und 9 Gew.-% $CF_3$-$CF_2$-$CF_2$-O-CF = $CF_2$ eingefüllt. Das zugeschmolzene Glasrohr wurde 3 Tage auf 150 °C erhitzt. Anschließend wurde vom Gemisch ein $^{19}$F-NMR-Spektrum mit $CFCl_3$ als externem Standard aufgenommen. Es konnte kein Vinylether mehr nachgewiesen werden. Als Hauptkomponente (> 70 Gew.-%) wurde $(CF_3)_2CF$-$CF_2$-COF mit Signalen bei +22,5 ppm (1F, - COF, m), - 74,6 ppm (6F, $CF_3$, m), - 114,8 ppm (2F, $CF_2$, m) und - 188,1 ppm (1F, CF, m) nachgewiesen.

**Beispiel 7**

Im Autoklaven des Beispiels 1 wurden 100 g Br-$CF_2$-$CF_2$-O-CF = $CF_2$ 7 Stunden auf 200 °C erhitzt.
Das Rohprodukt (96 g) wurde mit Hilfe der $^{19}$F-NMR-Spektroskopie untersucht.
Danach waren im Rohprodukt der Vinylether Br-$CF_2$-$CF_2$-O-CF = $CF_2$ und das Säurefluorid Br-$CF_2$-$CF_2$-$CF_2$-COF im Mengenverhältnis von 1:2 neben anderen, nicht zu identifizierenden Substanzen vorhanden.
Nach der Veresterung mit Methanol wurde der Ester Br-$CF_2$-$CF_2$-$CF_2$-$COOCH_3$ durch Destillation bei einem Siedepunkt von 73-76 °C/133 mbar isoliert.

**Beispiel 8**

Im Autoklaven des Beispiels 1 wurden 100 g H-$CF_2$-$CF_2$-$CF_2$-O-CF = $CF_2$ 24 Stunden auf 200 °C erhitzt.
Der Autoklaveninhalt wurde nach Abkühlen auf Zimmertemperatur mit 30 $cm^3$ Methanol verestert. Die Aufarbeitung des Produktgemisches erfolgte durch Waschen mit Wasser und Trocknen über $Na_2SO_4$.
Es wurden 98 g organische Flüssigkeit erhalten, die mit Hilfe der Gaschromatogaphie untersucht wurde.
Danach enthielt das Substanzgemisch 43 % H-$(CF_2)_4$-$COOCH_3$.

**Beispiel 9**

Im Autoklaven des Beispiels 1 wurden 100 g H-$(CF_2)_5$-O-CF = $CF_2$ 20 Stunden auf 250 °C erhitzt.
Die Aufarbeitung erfolgte gemäß Beispiel 5. Die Destillation ergab eine Ausbeute an H-$(CF_2)_6$-$COOCH_3$ von 33 g.
Der Siedepunkt des Esters betrug 107-108 °C/133 mbar.

**Beispiel 10**

In der Apparatur des Beispiels 7 wurden 44,6 g (0,1 mol)

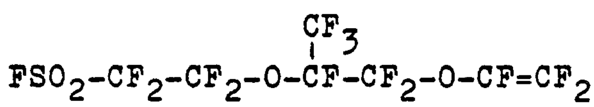

vorgelegt, die Apparatur mit Argon gespült und der Inhalt 12 Stunden unter Rückfluß (Innentemperatur 132-133 °C) erhitzt. Eine aus der Flüssigphase entnommene Probe zeigte im IR-Spektrum nur eine schwache Säurefluoridbande. Man setzte 50 ml des perfluorierten Polyethers

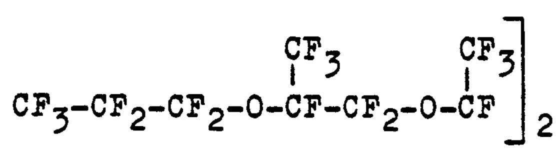

als inertes Lösemittel zu und erhitzte den Ansatz erneut 6 Stunden unter Rückfluß. Die Innentemperatur lag bei 170 °C. Danach war im IR-Spektrum einer Probe aus der Flüssigphase kein Vinylether mehr nachweisbar. Die Destillation lieferte bei einem Siedepunkt von 134-144 °C 17 g einer farblosen Flüssigkeit, die nach

dem $^{19}$F-NMR-Spektrum aus

$$FSO_2-CF_2-CF_2-O-\overset{CF_3}{\overset{|}{C}}F-CF_2-O-CF=CF_2 \quad \text{und}$$

$$FSO_2-CF_2-CF_2-O-\overset{CF_3}{\overset{|}{C}}F-CF_2-CF_2-COF$$

im Molverhältnis 1:2,2 bestand.

Die weitere Destillation ergab eine Fraktion (10 g) mit einem Siedepunkt von 144-213 °C, die nach dem $^{19}$F-NMR-Spektrum aus

$$FSO_2-CF_2-CF_2-O-\overset{CF_3}{\overset{|}{C}}F-CF_2-O-CF=CF_2 \ ,$$

$$FSO_2-CF_2-CF_2-O-\overset{CF_3}{\overset{|}{C}}F-CF_2-CF_2-COF \quad \text{und}$$

$$\left[CF_3-CF_2-CF_2-O-\overset{CF_3}{\overset{|}{C}}F-CF_2-O-\overset{CF_3}{\overset{|}{C}}F\right]_2$$

im Molverhältnis 1,5:5,2:1 bestand.

**Beispiel 11**

In einem Glaskolben, der mit einem Magnetrührer, Thermometer, Gaseinleitungsrohr und Rückflußkühler mit aufgesetztem Blasenzähler ausgestattet war, legte man 15 g

$$CH_3OOC-CF_2-CF_2-O-\overset{CF_3}{\overset{|}{C}}F-CF_2-O-CF=CF_2$$

vor und spülte die Apparatur mit Stickstoff. Der Ansatz wurde unter Rückfluß erhitzt. Dadurch stieg innerhalb von 8 Stunden die Sumpftemperatur von 159 °C auf 182 °C an. Vom Reaktionsgemisch wurde ein $^{19}$F-NMR-Spektrum aufgenommen, das einen Umsatz des Vinylethers von mehr als 80 % zeigte. Die Destillation ergab eine Fraktion (3 g) mit einem Siedepunkt von 49 °C/13 mbar, die nach dem $^{19}$F-NMR-Spektrum neben geringen Verunreinigungen die Verbindungen

$$CH_3OOC-CF_2-CF_2-O-\overset{CF_3}{\overset{|}{C}}F-CF_2-O-CF=CF_2$$

$$\text{und} \quad CH_3OOC-CF_2-CF_2-O-\overset{CF_3}{\overset{|}{C}}F-CF_2-CF_2-COF$$

im Molverhältnis 1:2,2 enthielt.

**Beispiel 12**

In der Apparatur des Beispiels 11 legte man 100 g des Perfluorpolyethers

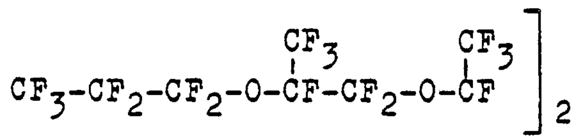

und 10 g $H-(CF_2)_3-O-CF-CF_2-O-CF=CF_2$ vor und erhitzte den Ansatz 1,5 Stunden auf ca. 180 °C Innentemperatur.
Anschließend wurde das Reaktionsgemisch destilliert, bis der Siedepunkt von ca. 200 °C erreicht war. Das Destillat (7,3 g) wurde mit Hilfe der IR-Spektroskopie untersucht. Neben einer sehr starken Bande für die COF-Gruppe die 1887 $cm^{-1}$ wurde nur noch eine sehr schwache Bande für die Vinylethergruppe bei 1840 $cm^{-1}$ erhalten.
Der Versuch wurde weiter geführt, in dem man den im Sumpf zurückgebliebenen Perfluorpolyether auf 170-190 °C erhitzte und im Laufe von 11 Stunden 95,3 g "H-PPVE-2" zutropfte. Gleichzeitig wurde das bei 115-120 °C siedende Gemisch abdestilliert. Man erhielt 83 g Destillat, das mit Methanol verestert, mit Wasser gewaschen und über $Na_2SO_4$ getrocknet wurde. Vom veresterten Produkt (80 g) wurde anschließend ein GC (WLD) aufgenommen. Es zeigte die Zusammensetzung:
59,0 % H-PPVE-2

32,4 % 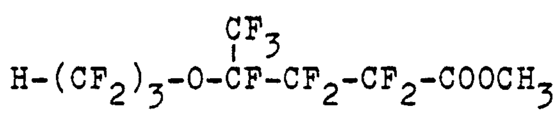

6,8 % Perfluorpolyether.
Durch Destillation wurde der Ester

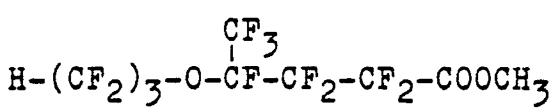

bei einem Siedepunkt von 85 °C/67 mbar isoliert.

**Ansprüche**

1. Verfahren zur Herstellung von fluorierten Carbonsäurefluoriden der Formel I

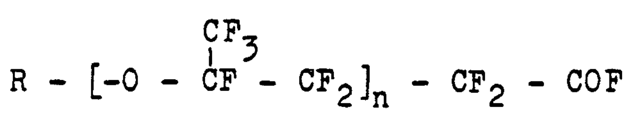 (I),

worin R ein verzweigter oder unverzweigter perfluorierter Rest mit 1-10 Kohlenstoffatomen bedeutet, in dem ein oder mehrere Fluoratome durch andere Halogenatome oder Wasserstoff oder eine andere funktionelle Gruppe substituiert sein können, und n eine ganze Zahl von null bis 10 ist, dadurch gekennzeichnet, daß ein Vinylether der Formel II

$$R - [O - CF(CF_3) - CF_2]_n - O - CF = CF_2 \qquad (II),$$

mit R und n gemäß Formel I, auf eine Temperatur von 100 bis 350 °C erhitzt wird.

2. Verfahren nach Anspruch 1, dadurch gekennzeichnet, daß die Reaktion in einem Temperaturbereich von 150 bis 350 °C durchgeführt wird.

Europäisches Patentamt

# EUROPÄISCHER RECHERCHENBERICHT

Nummer der Anmeldung

EP 89 11 3928

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | JOURNAL OF THE CHEMICAL SOCIETY 1968, Seiten 398-405, Chemical Society, London, GB; R.N. HASZELDINE et al.: "Perfluoroalkyl Derivatives of Nitrogen. Part XXVI. The Preparation and Rearangement of Polyfluorovinylamines and of Trifluotomethyl Trifluorovinyl Ether" * Seite 400, linke Spalte; Seite 403, rechte Spalte * | 1 | C 07 C 51/58<br>C 07 C 59/135<br>C 07 C 53/50 |
| D,A | EP-A-0 260 713 (AUSIMONT S.P.A.) * Anspruch 1 * | 1 | |
| A | SU-A- 144 480 (A. CHEBURKOV) * Beispiel * | 1 | |
| A | DE-A-1 668 546 (DU PONT DE NEMOURS AND CO.) * Anspruch 1 * | 1 | |
| A | PATENT ABSTRACTS OF JAPAN Band 11, Nr. 63 (C-406)(2510), 26. Februar 1987; & JP - A - 61 225 150 (NIPPON MEKTRON) 06.10.1986 | 1 | **RECHERCHIERTE SACHGEBIETE (Int. Cl.5)**<br>C 07 C 51/00<br>C 07 C 59/00<br>C 07 C 53/00 |

Der vorliegende Recherchenbericht wurde für alle Patentansprüche erstellt

| Recherchenort | Abschlußdatum der Recherche | Prüfer |
|---|---|---|
| BERLIN | 31-10-1989 | PROBERT C.L. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)